# EUROPEAN PATENT SPECIFICATION

(11) **EP 1 948 208 B1**
(45) Date of publication and mention of the grant of the patent: **18.10.2017**
(21) Application number: 06828874.5
(22) Date of filing: 27.10.2006
(51) Int. Cl.: A61K 36/23, A61K 45/06

(54) **USE OF COMPOUNDS FROM CENTELLA ASIATICA**
VERWENDUNG DER INHALTSSTOFFE VON CENTELLA ASIATICA
UTILISATION DE COMPOSÉS PROVENANT DE CENTELLA ASIATICA

(30) Priority: 09.11.2005 EP 05292384
(43) Date of publication of application: 30.07.2008
(73) Proprietor: Société d'Exploitation des Produits pour les Industries Chimiques, SEPPIC, 75321 Paris cedex 07 (FR)
(72) Inventor: SENE, Gérard, F-75013 Paris (FR); LOISEAU, Alain, F-64140 Arzacq (FR); LEPETIT, Jean-Christophe, F-92700 Colombes (FR)
(74) Representative: Conan, Philippe Claude
(86) International application number: PCT/EP2006/010355
(87) International publication number: WO 2007/054211

(56) References cited:
- WO-A-2004/062678
- WO-A1-2007/010023
- WO-A2-2005/034891
- LEPETIT J-C: "MADECASSOSIDE - IMMUNE REGULATION OF PSORIASIS-LIKE DISORDERS BY NATURAL ANTI-INFLAMMATORY ACTIVE INGREDIENT" SOFW-JOURNAL SEIFEN, OELE, FETTE, WACHSE, VERLAG FUR CHEMISCHE INDUSTRIE, AUGSBURG, DE, vol. 131, no. 4, April 2005 (2005-04), page 28,30,32,34, XP008060903 ISSN: 0942-7694
- BONNET G F: "TRAITEMENT DES CELLULITES LOCALISEES PAR LE MADECASSOL" PROGRES MEDICAL, SN, PARIS,, FR, vol. 102, no. 3, March 1974 (1974-03), pages 109-110, XP001030401 ISSN: 0033-0450
- KIMURA Y ET AL: "Agent useful as a skin external preparation, such as cosmetics, for preventing and treating organic diseases causing histopathological change of skin or mucous membrane, comprises a preset amount of Centella asiatica extract", WPI/THOMSON,, vol. 2004, no. 18, 26 February 2004 (2004-02-26), XP002490656,

## Description

The present invention relates to the use of compounds from Centella Asiatica in cosmetics, pharmaceuticals and food supplements for improving keratinocytes differentiation and for enhancing the epidermal functionality.

Centella asiatica, also known as Violette marronne (Reunion Island), as Gotu Kola or as Indian pennywort (India), or as Centella repanda (North America) and as Talapetraka (Madagascar), is a polymorphous herb and belongs to the family of Umbelliferae (Apiaceae), particularly to the Hydrocotyle subfamily that grows wild in pantropical moist and shady regions at an ideal altitude of 600 to 1200 m. Centella asiatica includes three varieties called Typica, Abyssinica and Floridana. It is known and used for healing, sedative, analgesic, antidepressant, antiviral and antimicrobial properties. The biological activity is enabled by active molecules from the triterpene series such as Asiaticoside (I), Madecassoside (under its 2 isomeric forms : madecassoside itself and terminoloside) (II), Asiatic acid (III) and Madecassic Acid (IV). They contribute to the natural defense of the plants against environmental aggression thanks to the anti-bacterial properties of the genins (III, IV), which are obtained by hydrolysis from the heterosidic reserve form (I, II).

Extracts containing these compounds are used in the pharmaceutical and cosmetic industry regarding skin care and skin diseases, respectively for treating wounds, scars and venous insufficiency and for dermis restoration and anti-inflammatory properties as disclosed in WO2004/062678.

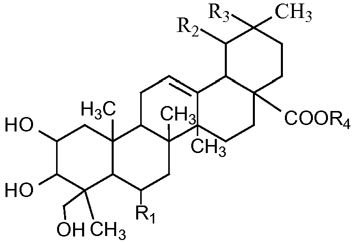

| | | R₁ | R₂ | R₃ | R₄ |
|---|---|---|---|---|---|
| I | Asiaticoside | H | CH₃ | H | Glu-Glu-Rha |
| II | Madecassoside | OH | CH₃ | H | Glu-Glu-Rha |
| | Terminoloside | OH | H | CH₃ | Glu-Glu-Rha |
| III | Asiatic Acid | H | CH₃ | H | H |
| IV | Madecassic Acid | OH | CH₃ | H | H |

Aquaporins were discovered in 1988. These transmembrane proteins serve to facilitate water transport through the skin down osmotic gradients with low activation energy. Eleven aquaporins have been identified in mammals to date. Aquaporin-3 (AQP-3) is unique in its structure (lowest homology with other aquaporins) and its function (Ishibashi, K; Sasaki, S; Fushimi, K; Yamamoto, T; Kuwahara, M; Marumo, F, The American Journal Of Physiology, 272 (2) 2, 1997, p. F235-F241). It is a glycerol and water transporter (aquaglyceroporin) regulated by p73 (Zheng, X; Chen, X, FEBS Letters, Volume 489, Issue 1, January 26, 2001, Pages 4-7). During development of the skin AQP-3 expression begins late in fetal life (Matsuzaki, T; Suzuki, T; Koyama, H; Tanaka, S; Takata, K, The Journal Of Histochemistry And Cytochemistry: Official Journal Of The Histochemistry Society, 47, (10), 1999, p. 1275-1286). Aquaporins serve as water channels and increase the permeability of membranes to water for as much as ten-fold. They are located in basal layers of epitheliums of numerous organs like kidney, lung or intestines (Takata K, Matzuaki T, Tajika Y, Prog Histem Cytochem 2004 (39) 1-835). Especially in the skin AQP-3 is located in keratinocytes (Sougrat R, Morand M, Gondran C, J Invest Dermatol 2002 (118) 678-85). The abundance of basolateral AQP-3 in epithelial tissue and its expression in many non-epithelial cells suggest that this aquaglyceroprotein is a major participant in osmolyte homeostasis in the human body and plays an important role in the hydration of the skin (Mobasheri, A; Wray, S; Marples, D, J Mol Histol, (36), 1-2, 2005, p. 1-14).

Several studies on mice deficient in AQP-3 demonstrate more directly the influence of AQP-3 for the state and functionality of the skin. According to Hara at al. (Hara, Mariko; Ma, Ronghui; Verkman AS, J of Biol Chem 277, 2002, (48), p 46616-46621) SHK1 mice have dry skin with reduced stratum corneum hydration, decreased elasticity and impaired functionality. Sougrat et al. propose AQP-3 as a water-clamp intended for improving the hydration of the epidermis below the stratum corneum (Sougrat R; Morand M; Gondran C; Barre, O; Gobin R; Bonte F; Dumas M; Verbavatz JM, J of Investigative Dermatology, 118 (4), 2002, 678-685). In a more recent study Hara et al. (Hara-Chikuma, Mariko; Verkman, A S, Biology Of The Cell / Under The Auspices Of The European Cell Biology Organization, (97), 7, 2005, p. 479-486) suggest that the glycerol rather than the water transporting function of AQP3 is important in skin physiology as the glycerol content of AQP-3 deficient mice is only reduced in the stratum corneum and in the epidermis. Accordingly the dry and relatively inelastic skin in AQP-3 null mice is probably related to the humectant properties of glycerol and the impaired stratum corneum repair due to impaired epidermal biosynthetic function.

Moreover AQP-3 is implicated in the triglyceride content of the epidermis and contributes partially to the lipid composition of the stratum corneum and ceramides. The water and associated transfer of electrolytes is beneficial for cells communication, reparation and drainage. Therefore AQP-3 regulating factors are helpful for the treatment of skin diseases associated with altered skin, water content, lipidic disorders and cells communication dysfunctions (WO 2001/37799).

Functionality of the epidermis can also be altered by an impairment of the stratum corneum organization and more generally by a poor differentiation of keratinocytes leading to a decrease of the skin barrier function. Genetic or chronic deficiencies on ceramides, (pro-)filaggrin, Natural Moisturizing Factor, Transglutaminase etc. can be origins for a poor differentiation and a deficient maturation of keratinocytes.

Filaggrin in particular is important in the organization of lamellar corneocytes and consequently in the maintenance of skin water content and/or of the biological and physical skin integrity. Filaggrin is a basic, histidine-rich protein synthesized by cells of keratinizing epithelia that support major physiological functions in epidermis. Its protein precursor profilaggrin constitutes a major component of keratohyalin granules in the epidermal granular layer (Kanitakis J, Ramirez-Bosca A, Reano A, Viac J, Roche P, Thivolet J. Virchows Arch A Pathol Anat Histopathol. 1988, 412(4), 375-82). Obtained by dephosphorylation of profilaggrin, the filaggrin filament-associated protein interacts with keratin to aggregate it in the stratum corneum of mammalian epidermis during the terminal differentiation of keratinocytes into corneocytes. It therefore plays a role in the organization of lamellar corneocytes and the cells cohesion, stratum corneum resistance and flexibility as well as in epidermal barrier function.

Another example for epidermal functionality key compounds concerns transglutaminases, which play in important role in mature keratinocytes. Transglutaminases (TGM) are a widely distributed group of calcium-dependent enzymes that catalyse formation of covalent isopeptide bonds, the obtained cross-linked products are highly resistant to mechanical challenge and proteolytic degradation (Biochem J, 368, 2002, 377-96). Four out of the nine TGM discovered to date are expressed in the epidermis. These enzymes have been shown to be expressed maximally in the upper spinous and granular cells of the epidermis (Cell, 40, 1985, 685-695; Differentiation, 33, 1986,130-141).

TGM are particularly essential in the terminal differentiation of the epidermis, where they heavily cross-link keratins and a range of differentiation-specific structural proteins (such as involucrin, loricrin, etc.), in the formation of the cornified cell envelope in the biogenesis of the stratum corneum (Bioessays, 24, 2002, 789-800; J. Cell Sc, 95, 1990, 631-638). Mutations of TGM and their substrates cause severe skin diseases: for example, dysfunction of TGM-1 have been shown to cause lamellar ichtyosis, a disease characterized by excessive scaling and shedding of the outer epidermis (Am. J. Hum.Genet., 77, 2005, 907-917; Nat Rev Mol Cell Biol, 6, 2005, 328-340).

The international application WO 2005/034891 A2 discloses a cosmetic composition having ingredients that may prevent signs of aging, improve the aesthetic appearance of skin, and promote recovery from environmental stresses. The composition includes natural ingredients, including at least one ingredient or extract from rosemary, at least one ingredient or extract from Centella, Echinacea, Alpinia or mixtures thereof, a DNA repair enzyme, and at least one pharmaceutically or cosmetically acceptable vehicle.

J.-C. LePetit discloses in SÖFW-journal A. 2005, vol. 131, no. 4, pp. 28-34 that to date, three of the four known triterpenes (asiaticoside, asiatic acid and madecassic acid) have been widely used in pharmacy and cosmetics, especially for their capacity to stimulate collagen synthesis and their dermis and venous wall restructuring properties. This use seemed to not only indicate a capacity for fibroblast activity stimulation, but also regulation, in the case of an inflammatory hyperproliferation responsible for the keloid formation. This anomaly oriented the research towards the study of the anti-inflammatory properties of Centella asiatica triterpenes and constitutes a new investigation area.

The present invention relates to compounds from Centella asiatica selected from the group consisting of madecassoside, terminoloside and asiaticoside for improving the differentiation of keratinocytes and for enhancing the epidermal functionality.

Subject of the present invention is the use of a mixture comprising madecassoside and terminoloside or a plant extract comprising a mixture of madecassoside and terminoloside for the manufacture of a composition for treating dysplasia, cutaneous squamous lesions, ichthyosis or ulceration of the skin, wherein the mixture ratio between madecassoside and terminoloside is from 30:70 up to 70:30 by weight when the mixture is used for the manufacture of a composition for treating ulceration.

The active compounds according to the invention are madecassoside, terminoloside and asiaticoside, and can be found e.g. in the plant Centella asiatica (e.g. from Madagascar). Extracts containing the active compounds of the present invention can be obtained e.g. by extraction of Centella asiatica as described in WO2004/062678. The extracts of Centella asiatica can comprise different pentacyclic triterpenes such as asiatic acid, asiaticoside, madecassic acid, madecassoside and/or terminoloside. The active compounds can be isolated from the extracts of Centella asiatica as described in WO2004/062678. The compounds of the present invention can be used as a mixture comprising madecassoside and terminoloside.

Subject of the present invention is also the use of a plant extract, preferably a plant extract of Centella asiatica, comprising a mixture comprising madecassoside and terminoloside.

Preference is given to a plant extract containing madecassoside and terminoloside and optionally asiaticoside with an amount of more than 75%, preferably more than 85% by weight of the total plant extract. Other impurities can be e.g. fatty acids. The ratio asiaticoside: (madacasso-side+terminoloside) can be from 5:95 up to 25:75. The ratio of madecassoside and terminoloside can be from 30:70 up to 70:30, preferably 40:60 up to 60:40 by weight.

The solvent of the mixture is preferably a mixture of water and alcohol e.g. ethanol. The ratio of the volume between water and alcohol can be from 50:50 up to 90:10, preferably 75:25.

The ratio of madecassoside and terminoloside can be from 30:70 up to 70:30, more preferably 40:60 up to 60:40 by weight. The purity of the mixture is preferably greater than 95% relative to the total weight of the mixture. More preferably the mixture contains no or only in trace amounts of asiaticoside.

Mixtures and extracts of the present invention can be administered in any form by any effective route, including, e.g., oral, parenteral, enteral, intravenous, intraperitoneal, topical, transdermal (e.g., using any standard patch), ophthalmic, nasally, local, non-oral, such as aerosol, inhalation, subcutaneous, intramuscular, buccal, sublingual, rectal, vaginal, intra-arterial, and intrathecal, etc. They can be administered alone, or in combination with any ingredient(s), active or inactive. Preference is given to a topical administration.

Compounds, mixtures and extracts of the present invention can be converted in a known manner into the usual formulations such as cosmetic or pharmaceutical compositions or compositions used as food supplement. These may be liquid or solid formulations e.g. without limitation normal and enteric coated tablets, capsules, pills, powders, granules, elixirs, tinctures, solution, suspensions, suppositories, syrups, solid and liquid aerosols, emulsions, pastes, creams, ointments, milks, gels, salves, serums, foams, shampoos, sticks or lotions.

Preference is given to a cosmetic composition in a form of an aqueous solution, a white or colored cream, ointment, milk, gel, salve, serum, foam, shampoo, stick, cream, paste, or lotion.

Mixtures and extracts of the present invention can be further combined with any other suitable additive or pharmaceutically acceptable carrier. Such additives include any of the substances already mentioned, as well as any of those used conventionally, such as those described in Remington: The Science and Practice of Pharmacy (Gennaro and Gennaro, eds, 20th edition, Lippincott Williams & Wilkins, 2000); Theory and Practice of Industrial Pharmacy (Lachman et al., eds., 3rd edition, Lippincott Williams & Wilkins, 1986); Encyclopedia of Pharmaceutical Technology (Swarbrick and Boylan, eds., 2nd edition, Marcel Dekker, 2002). These can be referred to herein as "pharmaceutically or cosmetically acceptable carriers" to indicate they are combined with the active drug and can be administered safely to a subject for therapeutic purposes.

The dosage of the compounds, mixtures and extracts of the present invention can be selected with reference to the other and/or the type of disease and/or the disease status in order to provide the desired therapeutic activity. These amounts can be determined routinely for a particular patient, where various parameters are utilized to select the appropriate dosage (e.g., type of disease, age of patient, disease status, patient health, weight, etc.), or the amounts can be relatively standard.

The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

Preference is given to a composition comprising the active compounds, mixtures and extracts of the present invention in an amount of from 0.005% up to 10%, preferably 0.01 up to 5%, more preferably 0.1% up to 3% by weight of the total composition.

The pharmaceutical or cosmetic composition according to the invention is administered one or more, preferably up to three, more preferably up to two times per day. Preference is given to a topical administration.

Nevertheless, it may in some cases be advantageous to deviate from the amounts specified, depending on body weight, individual behaviour toward the active ingredient, type of preparation and time or interval over which the administration is effected. For instance, less than the aforementioned minimum amounts may be sufficient in some cases, while the upper limit specified has to be exceeded in other cases. In the case of administration of relatively large amounts, it may be advisable to divide these into several individual doses over the day.

Administration of the mixtures or extracts of the present invention can increase the expression of AQP-3 in the epidermis, preferably in the living layers (basal keratinocytes, stratum granulosum & spinosum), the expression of filaggrin and/or of transglutaminase. Therefore mixtures or extracts of the present invention can be used for any disease or disorder associated with a deficiency of AQP-3, filaggrin and/or transglutaminase expression. When the mixtures or extracts of the present invention are administered e.g. the hydration of the skin, preferably hydration of the epidermis below the stratum corneum, can be maintained or improved. Also the elasticity and/or epidermal resistance of the skin can be maintained or restored. The lamellar organization of corneocytes can be improved and the cohesion of stratum corneum, epidermal barrier function and/or skin impermeability can be increased. Epidermal dehydration can be prevented and the regeneration of the protective lipid film of the skin can be supported. The electrolyte and water transfer of the skin can be improved. Osmolytic homeostatis can be activated and/or regenerated. The epidermal detoxification, the communication of keratinocytes and/or the reparation of the membrane of keratinocytes can be improved. Furthermore the drainage of keratinocytes can be enhanced.

Mixtures and extracts of the present invention can be used for improving the differentiation of keratinocytes, for enhancing the epidermal functionality and/or for treating or preventing of skin diseases or skin disorders such as ulceration, dysplasia, cutaneous squamous lesionsor ichthyosic skins.

Mixtures and extracts of the present invention can also be used for the treatment and/or prevention of abnormalities of epidermal maturation and/or keratinisation.

Preferably mixtures and extracts of the present invention are used for activating AQP-3 e.g. in different epidermal layers. Therefore the transfer of water, water-soluble compounds such as electrolytes or neutral solutes, and/or the transfer of glycerol can be increased which result in activation or regeneration of osmolyte homeostasis, enhancement of the keratinocytes drainage and so improvement of the subsequent cell detoxification, keratinocytes membrane reparation and lipidic disorders treatment, improvement of the keratinocytes communication (between cells and between epidermis on the one hand and the Epidermal Dermal Junction or the dermis on the other hand).

Preferably compounds, mixtures and extracts of the present invention are used for activating filaggrin whereby the activity on the differentiation of keratinocytes is affected. Therefore abnormalities of epidermal maturation and/or keratinisation can be treated, cohesion of stratum corneum (corneocytes lamellar organization), the epidermal barrier function and/or skin impermeability is increased, epidermal dehydration can be prevented, and the epidermal elasticity/flexibility and resistance is improved.

Preferably compounds, mixtures and extracts of the present invention are used for activating transglutaminase whereby the activity on the maturation of keratinocytes is affected. Therefore abnormalities of epidermal maturation and/or keratinisation can be treated, cohesion of stratum corneum (corneocytes lamellar organization), the epidermal barrier function and/or skin impermeability is increased and the epidermal resistance is improved.

### Examples:

### Example 1: Evaluation of keratinocytes AQP-3 expression

Biopsies from abdominal plastic surgery (41-year-old woman) are used in this ex vivo experiment. They are cultured in a specific survival explants medium: BEM (BIO-EC's Explants Medium).

4 mg of a formulation containing 3% of a mixture of madecassoside and terminoloside is applied versus a control formulation (only excipient without active compounds) at the following days: D0, D1, D2, D3 and D4.

Histological studies are performed at the following after 0 h, 3 h, 24 h and 5 days.

After dehydration and paraffin impregnation, explants are fixed with Bouin's solution. They are then cut and stained by Masson's trichome stain.

Flurorescent immunomarking: Aquaporin-3 (AQP-3) is marked on frozen cryostat cut tissues with anti-AQP3 from Chemicon (polyclonal ref AB3276) with a biotin/streptavidin system and revealed by FITC. Observations are done by electronic microscope and quantification by image analysis. They are quantified as followed:

### Observations of the expression of AQP 3 in the epidermic layers

| time | 0h | 3h | 24h | 5 days |
|---|---|---|---|---|
| Stratum corneum basis | | | | |
| Control | 0 | | | |
| Excipient | | 0 | 0 | 3 |
| Excipient + 3% mixture | | 0 | 1 | 4 |

| Basal poles of basal keratinocytes | | | | |
|---|---|---|---|---|
| Control | 0 | | | |
| Excipient | | 0 | 0 | 3 |
| Excipient + 3% mixture | | 0 | 4 | 4 |

Absent: 0; weak: 1, very moderate: 2, moderate: 3, clear: 4, very clear: 5, high: 6
At 0 h, the AQP-3 expression is clearly visible in membranes (regular, pericellular presence). It is absent at the base of the stratum corneum, clear-cut in the epidermal upper layers, moderate in the basal layer and absent in the basal pole of basal keratinocytes (usually AQP-3 lacking area).
A time-dependent phenomenon is observed for the increase of AQP-3 expression in epidermis by administration of the mixture: AQP-3 is more clearly visualized after 3h, and even more after 24h. After 5 days an important development of the protein channels is observed at the basal pole of the basal keratinocytes. This particular location of AQP-3 is likely a favourable factor for the dermoepidermal communication, especially for the heme originally from vascularization, which is a known differentiation inductor.

### Quantification of the expression of AQP 3 in the epidermic layers

Percentage of the surface occupied by the aquaporin 3 at day 5

| | In the living epidermis | | In the basal pole of the basal keratinocytes | |
|---|---|---|---|---|
| | Average | Deviation | Average | Deviation |
| Excipient | 42,9 | 1,9 | 31,1 | 1,8 |
| Excipient+3% mixture | 56,9 | 3,1 | 44,9 | 3,7 |

On day 5 and by comparison to the excipient, the formulation containing 3% of a mixture Madecassoside - Terminoloside significantly increases the AQP-3 content by 33% in the living epidermis and by 44% in the basal pole of the basal keratinocytes layer.

### Example 2: Evaluation of keratinocytes Filaggrin synthesis

Biopsies from abdominal plastic surgery (41-year-old woman) are used in this ex vivo experiment. They are cultured in a specific survival explants medium: BEM (BIO-EC's Explants Medium).

4 mg of a formulation containing 3% of a mixture of madecassoside and terminoloside is applied versus a control formulation (only excipients without active compounds) at the following days: D0, D1, D2, D3 and D4.

Histological studies are performed after 0 h, 3 h and 24 h.

After dehydration and paraffin impregnation, explants are fixed with Bouin's solution. They are then cut and stained by Masson's trichome stain.

Fluorescent immunomarking: Filaggrin is marked on frozen cryostat cut tissues with anti-filaggrin from BTI Cliniscience (clone OKTB1 ref BT 576) with a biotin/streptavidin system and revealed by FITC. Observations are done by electronic microscope and quantification by image analysis.

### Observations of the expression of filaggrin in the epidermic layers

At 0 h filaggrin is normally expressed at the stratum corneum basis. After 3 h an increased expression is observed. After 24 h an increased fluorescence is observed in a greater number of cells layers (marking present in 9 to 10 layers from the stratum corneum basis).

The cohesion of the stratum corneum stratification is improved due to filaggrin. Its increased concentration in the upper granular layer leads to an enhancement of the organization and physical quality in the stratum corneum (apoptotic regulation) and a strengthening of the hydric affinity following its hydrolysis. Therefore the anti-dryness activity can be improved.

### Quantification of the expression of filaggrin in the epidermic layers

Percentage of the surface occupied by filaggrin in the epidermis at after 24h

| | t = 0 h | | t = 24h | |
|---|---|---|---|---|
| | Average | Deviation | Average | Deviation |
| Blank | 9,8 | 1,4 | | |
| Excipient | | | 5,9 | 1,8 |
| Excipient + 3% mixture | | | 9,4 | 1,7 |

On day 1 and by comparison to the excipient, the formulation containing 3% of a mixture Madecassoside - Terminoloside significantly increases the filaggrin content of epidermis by 59%.

### Example 3: Evaluation of keratinocytes transglutaminase synthesis

Biopsies from abdominal plastic surgery (41-year-old woman) are used in this ex vivo experiment. They are cultured in a specific survival explants medium: BEM (BIO-EC's Explants Medium).

4 mg of a formulation containing 3% of a mixture of madecassoside and terminoloside is applied versus a control formulation (only excipients without active compounds) at the following days: D0, D1, D2, D3 and D4.

Histological studies are performed after 0 h, 3 h and 24 h.

After dehydration and paraffin impregnation, explants are fixed with Bouin's solution. They are then cut and stained by Masson's trichome stain.

Fluorescent immunomarking: Transglutaminase is marked on frozen cryostat cut tissues with anti-membrane transglutaminase from Harbor Bio-products (MAB Clone B.C1) with a biotin/streptavidin system and revealed by FITC. Observations are done by electronic microscope and quantification by image analysis.

### Observations of the expression of transglutaminase in the epidermic layers

On day0, the transglutaminase expression is light and irregular.
After 24 hours, the immunostaining is clear on explants receiving 3% of the Madecassoside/Terminoloside blend. It is clearly lighter on thoses receiving the excipient.

### Quantification of the expression of transglutaminase in the epidermic layers

Percentage of the surface occupied by transglutaminase in the epidermis after 24h

| | t = 0 h | | t = 24 h | |
|---|---|---|---|---|
| | Average | Deviation | Average | Deviation |
| Blank | 0,73 | 0,51 | | |
| Excipient | | | 0,02 | 0,02 |
| Excipient + 3% mixture | | | 0,20 | 0,14 |

On day 1 and by comparison to the excipient, the formulation containing 3% of a mixture Madecassoside - Terminoloside moderately but significantly increases the transglutaminase.

### Example 4: Typical composition of a cream

### Cream 1:

Ingredients (INCI, w/w%): 1.0% of active compound (e.g. mixture of madecassoside and terminoloside, or extract of Centella asiatica according to the invention), 1.5% of beheneth-10, 1.5% of beheneth-25, 5.0% of dycaprylyl carbonate, 5.0% of hexyl laurate, 5.0% of isohexadecane, 5.0% of cetearyl isononaoate, 1.0% of dimethicone, 2.0% of behenyl alcohol, 2.0% of hydrogenated vegetable glycerides, 0.5% of phenoxyethanol and parabens, 0.5% of tocopherol acetate, 3.0% of glycerol, 2.0% butylenes glycol, 0.1% of xanthan gum, 0.2% of carbomer and water (qs 100).

### Cream 2:

Ingredients (INCI, w/w%): 1.0% of active compound (e.g. mixture of madecassoside and terminoloside, or extract of Centella asiatica according to the invention), 5.0% of cetearyl glucoside and cetearyl alcohol, 5.0% of caprylic/capric triglyceride, 5.0% of squalane, 3.0% of cetearyl isononaoate, 2.0% of dimethicone crosspolymer, 1.5% of stearyl alcohol, 5,0% of dycaprylyl carbonate, 0.1% parabens, 0.5% of phenoxyethanol and parabens, 2.0% of glycerol, 0.3% of carbomer, 2.0% of PEG 32, 0.2% of xanthan gum, 2.0% of aluminium starch octenyl succinate and water (qs 100).

## Claims

1. Use of a mixture comprising madecassoside and terminoloside or a plant extract comprising a mixture of madecassoside and terminoloside for the manufacture of a composition for treating dysplasia, cutaneous squamous lesions or ichtyosis of the skin.

2. Use of a mixture comprising madecassoside and terminoloside or a plant extract comprising a mixture of madecassoside and terminoloside for the manufacture of a composition for treating ulceration of the skin, wherein the mixture ratio between madecassoside and terminoloside is from 30:70 up to 70:30 by weight.

3. Use according to claim 1, wherein the mixture ratio between madecassoside and terminoloside is from 30:70 up to 70:30 by weight.

4. Use according to any of claims 1 to 3, wherein the plant extract is an extract of Centella asiatica.

5. Use according to any of claims 1 to 4, wherein the plant extract contains a mixture of madecassoside and terminoloside in an amount of more than 75% by weight of the total extract.

6. Use according to any of claims 1 to 4, wherein the plant extract contains a mixture of madecassoside and terminoloside and asiaticoside in an amount of more than 75% by weight of the total extract.

7. Use according to any of claims 1 to 6, wherein the solvent of the extract is a mixture of water and ethanol.

8. Use according to any of claims 1 to 7, whereby Aquaporin-3 (AQP-3), filaggrin and/or transglutaminase are activated.

9. Use according to any of claims 1 to 8 for increasing the transfer of water, water-soluble compounds and/or glycerol.

10. Use according to any of claims 1 to 9 for activation or regeneration of osmolyte homeostasis, enhancement of the keratinocytes drainage and so improvement of the subsequent cell detoxification, keratinocytes membrane reparation and lipidic disorders treatment, improvement of the keratinocytes communication.

11. Cosmetic composition comprising a mixture comprising madecassoside and terminoloside or a plant extract comprising a mixture of madecassoside and terminoloside for use in the treatment of dysplasia, cutaneous squamous lesions or ichtyosis of the skin.

12. Cosmetic composition comprising a mixture comprising madecassoside and terminoloside or a plant extract comprising a mixture of madecassoside and terminoloside for use in the treatment of ulceration of the skin, wherein the mixture ratio between madecassoside and terminoloside is from 30:70 up to 70:30 by weight.

13. Cosmetic composition for use according to claim 11, wherein the mixture ratio between madecassoside and terminoloside is from 30:70 up to 70:30 by weight.

14. Cosmetic composition for use according to claim 11 to 13, wherein the plant extract is an extract of Centella asiatica.

## Patentansprüche

1. Verwendung einer Mischung, umfassend Madecassosid und Terminolosid oder einen Pflanzenextrakt, umfassend eine Mischung aus Madecassosid und Terminolosid zur Herstellung einer Zusammensetzung zur Behandlung von Dysplasie, Hautepithelläsion oder Ichthyose der Haut.

2. Verwendung einer Mischung, umfassend Madecassosid und Terminolosid oder einen Pflanzenextrakt, umfassend eine Mischung aus Madecassosid und Terminolosid zur Herstellung einer Zusammensetzung zur Behandlung der Geschwürbildung der Haut, wobei das Mischverhältnis zwischen Madecassosid und Terminolosid von 30:70 bis 70:30 nach Gewicht liegt.

3. Verwendung nach Anspruch 1, wobei das Mischverhältnis zwischen Madecassosid und Terminolosid von 30:70 bis 70:30 nach Gewicht liegt.

4. Verwendung nach einem der Ansprüche 1 bis 3, wobei der Pflanzenextrakt ein Extrakt aus *Centella asiatica* ist

5. Verwendung nach einem der Ansprüche 1 bis 4, wobei der Pflanzenextrakt eine Mischung aus Madecassosid und Terminolosid in einer Menge von mehr als 75 Gew.% des gesamten Extrakts enthält.

6. Verwendung nach einem der Ansprüche 1 bis 4, wobei der Pflanzenextrakt eine Mischung aus Madecassosid und Terminolosid und Asiaticosid in einer Menge von mehr als 75 Gew.% des gesamten Extrakts enthält.

7. Verwendung nach einem der Ansprüche 1 bis 6, wobei das Lösemittel des Extrakts eine Mischung aus Wasser und Ethanol ist.

8. Verwendung nach einem der Ansprüche 1 bis 7, wobei Aquaporin-3 (AQP-3), Filaggrin und/oder Transglutaminase aktiviert sind.

9. Verwendung nach einem der Ansprüche 1 bis 8, zur Erhöhung des Transfers von Wasser, wasserlöslichen Verbindungen und/oder Glycerol.

10. Verwendung nach einem der Ansprüche 1 bis 9, zur Aktivierung oder Regenerierung von Osmolythomöostase, Verbesserung der Drainage der Keratinozyten und somit Verbesserung der folgenden Zellentgiftung, Reparatur der Membrane der Keratinozyten und Behandlung von Lipidstörungen, Verbesserung der Kommunikation der Keratinozyten.

11. Kosmetische Zusammensetzung, umfassend eine Mischung, umfassend Madecassosid und Terminolosid oder einen Pflanzenextrakt, umfassend eine Mischung aus Madecassosid und Terminolosid zur Verwendung bei der Behandlung von Dysplasie, Hautepithelläsion oder Ichthyose der Haut.

12. Kosmetische Zusammensetzung, umfassend eine Mischung, umfassend Madecassosid und Terminolosid oder einen Pflanzenextrakt, umfassend eine Mischung aus Madecassosid und Terminolosid zur Verwendung bei der Behandlung der Geschwürbildung der Haut, wobei das Mischverhältnis zwischen Madecassosid und Terminolosid von 30:70 bis 70:30 nach Gewicht liegt.

13. Kosmetische Zusammensetzung zur Verwendung nach Anspruch 11, wobei das Mischverhältnis zwischen Madecassosid und Terminolosid von 30:70 bis 70:30 nach Gewicht liegt.

14. Kosmetische Zusammensetzung zur Verwendung nach Anspruch 11 bis 13, wobei der Pflanzenextrakt ein Extrakt aus *Centella asiatica* ist.

## Revendications

1. Utilisation d'un mélange comprenant du madécassoside et du terminoloside ou d'un extrait végétal comprenant un mélange de madécassoside et de terminoloside pour la fabrication d'une composition destinée au traitement d'une dysplasie, des lésions cutanées squameuses ou de l'ichtyose de la peau.

2. Utilisation d'un mélange comprenant du madécassoside et du terminoloside ou d'un extrait végétal comprenant un mélange de madécassoside et de terminoloside pour la fabrication d'une composition destinée au traitement d'une ulcération de la peau, dans laquelle le rapport dans le mélange entre le madécassoside et le terminoloside est de 30:70 jusqu'à 70:30 en poids.

3. Utilisation selon la revendication 1, dans laquelle le rapport dans le mélange entre le madécassoside et le terminoloside est de 30:70 jusqu'à 70:30 en poids.

4. Utilisation selon l'une quelconque des revendications 1 à 3, dans laquelle l'extrait végétal est un extrait de Centella asiatica.

5. Utilisation selon l'une quelconque des revendications 1 à 4, dans laquelle l'extrait végétal contient un mélange de madécassoside et de terminoloside dans une quantité de plus de 75 % en poids de l'extrait total.

6. Utilisation selon l'une quelconque des revendications 1 à 4, dans laquelle l'extrait végétal contient un mélange de madécassoside et de terminoloside et d'asiaticoside dans une quantité de plus de 75 % en poids de l'extrait total.

7. Utilisation selon l'une quelconque des revendications 1 à 6, dans laquelle le solvant de l'extrait est un mélange d'eau et d'éthanol.

8. Utilisation selon l'une quelconque des revendications 1 à 7, par laquelle l'Aquaporine-3 (AQP-3), la filaggrine et/ou la transglutaminase sont activées.

9. Utilisation selon l'une quelconque des revendications 1 à 8 pour l'augmentation du transfert de l'eau, des composés hydrosolubles et/ou du glycérol.

10. Utilisation selon l'une quelconque des revendications 1 à 9 pour l'activation ou la régénération de l'homéostasie des osmolytes, l'amplification du drainage des kératinocytes et ainsi l'amélioration de la détoxification cellulaire subséquente, la réparation de la membrane des kératinocytes et le traitement des troubles lipidiques, l'amélioration de la communication des kératinocytes.

11. Composition cosmétique comprenant un mélange comprenant du madécassoside et du terminoloside ou un extrait végétal comprenant un mélange de madécassoside et de terminoloside pour une utilisation dans le traitement d'une dysplasie, des lésions cutanées squameuses ou de l'ichtyose de la peau.

12. Composition cosmétique comprenant un mélange comprenant du madécassoside et du terminoloside ou un extrait végétal comprenant un mélange de madécassoside et de terminoloside pour une utilisation dans le traitement d'une ulcération de la peau, dans laquelle le rapport dans le mélange entre le madécassoside et le terminoloside est de 30:70 jusqu'à 70:30 en poids.

13. Composition cosmétique pour une utilisation selon la revendication 11, dans laquelle le rapport dans le mélange entre le madécassoside et le terminoloside est de 30:70 jusqu'à 70:30 en poids.

14. Composition cosmétique pour une utilisation selon les revendications 11 à 13, dans laquelle l'extrait végétal est un extrait de Centella asiatica.
